Europäisches Patentamt

European Patent Office

Office européen des brevets

(19)

(11) Publication number : **0 383 462 B1**

# EUROPEAN PATENT SPECIFICATION

(12)

(45) Date of publication of patent specification :
27.04.94 Bulletin 94/17

(51) Int. Cl.⁵ : **A61K 35/78**

(21) Application number : **90301118.7**

(22) Date of filing : **02.02.90**

(54) **Preparation of a medicament containing mustard.**

(30) Priority : **16.02.89 GB 8903533**

(43) Date of publication of application :
**22.08.90 Bulletin 90/34**

(45) Publication of the grant of the patent :
**27.04.94 Bulletin 94/17**

(84) Designated Contracting States :
**AT BE CH DE DK FR IT LI LU NL SE**

(56) References cited :
DE-A- 3 045 845
DE-B- 1 951 822
MEDLINE abstract no. 86226429; B. FISHER et al.: "Ten-year results from the national surgical adjuvant breast and bowel project (NSABP) clinical trial evaluating the use of L-phenylalanine mustard (L-PAM) in the management of primary breast cancer" & J. Clin. Oncol. June 1986, vol. 4, no. 6, pages 929-941

(56) References cited :
MEDLINE abstract no. 87102617; R.J. BERNACKI et al.: "Combinations of mesna with cyclophosphamide or adriamycin in the treatment of mice with tumors" & Cancer Res. February 1987, vol. 47, no. 3, pages 799-802
CHEMICAL ABSTRACTS vol. 98, no. 21, 23 May 1983, page 33, abstract no. 191378k, Columbus, Ohio, US; R.F. STRUCK et al.: "Isophosphoramide mustard, a metabolite of ifosfamide with activity against murine tumors comparable to cyclophosphamide" & Br. J. Cancer 1983, vol. 47, no. 1, pages 15-26

(73) Proprietor : **RECKITT AND COLMAN PRODUCTS LIMITED**
One Burlington Lane
London W4 2RW (GB)

(72) Inventor : **Dettmar, Peter Williams**
Willow House
Main Street, Welwick, HU12 0RY (GB)
Inventor : **LLyoyd-Jones, John Gareth**
17 Arras Drive
Cottinghham, North Humberside, HU16 5LE (GB)

(74) Representative : **Allard, Susan Joyce et al**
BOULT, WADE & TENNANT 27 Furnival Street
London EC4A 1PQ (GB)

EP 0 383 462 B1

Jouve, 18, rue Saint-Denis, 75001 PARIS

EP 0 383 462 B1

## Description

This invention relates to the preparation of a medicament for use in treatment to prevent the development of cancer of the bowel.

In Western Europe and North America the incidence of colorectal cancer is high with about 15% of all cancers located in the large bowel. Adenomatous polyps are considered to be the precursors of the carcinomas found in the large bowel and the prognosis still remains relatively poor. Most epidemiologists conclude that diet is the enviromental factor of most importance in colorectal cancer but attempts to identify the dietary components implicated have proved difficult although it has been suggested that dietary fibre may play a protective role in the aetiology of colorectal carcinogenesis.

Mustard Husk is a vegetable fibre and we have investigated its pharmacological proprerties.

Mustard, widely used as a flavouring agent for food, is derived from the seeds of certain plants of the Cruciferae family. There are two principal types of mustard; brown mustard obtained mainly from Brassica nigra and white (often called yellow) mustard obtained principally from Sinapis alba. White mustard is a native annual plant of Southern Europe but is now widely cultivated in many temperate countries including the UK. Mustard is used by the food industry in several forms: whole seed, ground seed meal, mustard cake (ground mustard seed from which a portion of the fixed oil has been expressed), mustard flour (ground mustard cake with hulls removed) and prepared mustard.

Mustard husk consists mainly of seed coats of mustard. It is prepared by milling of the whole seeds and separation of the seed coats by a winnowing process involving vibration in a controlled airstream. The husk is then milled and sieved to give a product of standardised particle size.

In our investigations of mustard husk we have used material derived from mustard seeds by a dry process not involving any chemical treatment of either the seeds or the separated outer shell from the seeds. Reference hereafter to untreated mustard husk is to be understood to refer to material separated from the kernel by a dry process that has not undergone any chemical or solvent extraction treatment.

According to this invention there is provided the use of untreated mustard husk for the preparation of a medicament for the prevention of colonic cancer.

The mustard husk to be used may be either white mustard husk or brown mustard husk. Preferably the husk is milled.

The medicament will for example be in the form of powders, granules, tablets, capsules or a suspension.

For ease of administration and for accuracy in dosing, the medicament may be prepared in unit dosage forms. Thus in the case of powders or granules they may be conveniently packaged into sachets, each containing a single dose, from 0.5 to 5g and especially 1.0 to 3.0g mustard husk. In the case of tablets or capsules each will contain from 0.5 to 1.0g of mustard husk with a single dose comprising one or more units as appropriate.

When preparing dosage forms heating should be avoided as this may lead to loss of activity of the mustard husk.

The medicaments in the form of granules may be prepared by standard methods such as dry granulation (slugging). They may be effervescent or non-effervescent to be mixed with a suitable quantity of water for administration as a drink. They may be chewable granules.

The medicaments in the form of tablets may be prepared by standard methods such as direct compression. The tables may be coated with suitable film-forming agents such as hydroxypropylmethylcellulose using standard techniques.

The medicaments in the form of capsules may be prepared by standard methods involving blending the mustard husk with conveniental diluents and filling into hard capsules.

The medicaments in the form of suspensions are prepared by mixing the components with water. In order to protect the suspensions against microbial deterioration it is preferable to include a preservative. A suitable system is a combination of methyl- and propyl- p-hydroxybenzoate (methyl paraben and propyl paraben).

The medicaments may also include one or more of a colourising, sweetening or flavouring agent.

The invention is illustrated by the following examples, in all of which untreated white mustard husk, milled, is employed. Brown mustard husk, milled, may similarly be employed.

EXAMPLE 1

Chewable granules

| | |
|---|---|
| White mustard husk, milled | 1000mg |
| Sorbitol BP | 1500mg |

2

| Magnesium stearate BP | 25mg |
|---|---|

<div style="text-align: center">

Colour )<br>
       )                                     as required<br>
Flavour)

</div>

The materials are blended and compressed using suitable equipment. The resulting compacts are broken into granules using suitable equipment, and the granules screened.

EXAMPLE 2

Direct compression tablet

| White mustard husk, milled | 500mg |
|---|---|
| Microcrystalline cellulose NF | 300mg |
| Compressible sugar NF | 100mg |
| Magnesium stearate BP | 5mg |

The mustard husk is blended with the excipients. The resultant mix is compressed into tablets using a suitable tablet press fitted with 11mm flat, bevelled-edge punches.

EXAMPLE 3

Capsules

| White mustard husk, milled | 500mg |
|---|---|
| Pregelatinised maize starch BP | 100mg |
| Magnesium stearate BP | 6mg |

The ingredients are blended and filled into size No 0 hard gelatin capsules using suitable equipment.

The ability of a mustard husk diet to modify chemically induced rat intestinal carcinogenesis after exposure to the carcinogen (ie during the promotional phase of carcinogenesis) was determined by the method of Wilpart, M and Roberfroid, M. Nutr. Cancer 10, 39-51 (1987).

In the test method six-week old male Wistar rats centraal poefdierenbedrijf (Technische Nederlandse Onderzoek, Utrecht, The Netherlands) were used. They were housed in pairs in suspended stainless steel cages under temperature and humidity controlled conditions with a 12:12-hour light-dark cycle. Food and water were available ad libitum. All animals were acclimatised to the experimental rat room for two weeks before the start of the experiment. All the animals were subcutaneously injected with neutralised (pH 7.2) EDTA ($10^{-3}$M) solution of the carcinogen 1,2-dimethylhydrazine (DMH), one of the most widely used carcinogens for the induction of intestinal carcinogenesis in experimental animals. The DMH was injected weekly for 15 successive weeks, at a dose of 30mg DMH hydrochloride per kilogram of adjusted body weight.

During this period the rats received a diet corrected for isocaloricity ie low fat lipid diet (5 % wt/wt).

The fat was a mixture of corn oil and palm oil (50:50) containing equal proportions of saturated, monounsaturated, and polyunsaturated fatty acids. This diet was considered the reference diet because it contained 5% of cis-cis linoleic acid, which is the minimal amount required for a regular growth of the rat. The first dose of DMH was given two weeks after the dietary protocol was begun.

After ceasing administration of DMH (Week 16) the animals were randomised and divided into four groups of 40 animals. They were given one of four specific diets containing either 0% fibre (control group) or dietary fibre as untreated milled white mustard husk added at 2.5, 5.0 or 7.5% in weight as a constituent of a high (20% wt/wt) fat lipid isocaloric diet. The rats remained on these diets for the remainder of the experiment.

Body weight and average food consumption was monitored every week and the faecal outflow was determined during the administration of the specific fibre diets.

Animals were observed daily from week 20 on unless they showed dramatic signs of illness. Surviving animals were killed (10 rats per group) at Week 26, Week 30 and Week 34 (from 1st DMH injection). A complete necropsy was performed on each animal. A longitudinal incision was made along the entire intestinal tract. The colon was cleared and the number, position, size and shape of intestinal tumours were noted. The tumours, major organs and all tissues with abnormalities were fixed in 10% neutral-buffered formalin and then embedded in paraffin for routine histological analysis using haematoxylin and eosin staining.

The influence of the specific diets on the small and large bowel and on the total intestinal (small and large bowel) carcinogenesis was analysed.

The global tumorigenic effects observed following the four specific diets in rats with tumours induced by the carcinogen DMH are illustrated in Tables 1 and 2.

Table 1 presents test data demonstrating the influence of untreated milled white mustard husk on the incidence of intestinal and colonic tumours in DMH-treated rats.

## TABLE 1

| Experimental Groups | No of rats* bearing tumours (% incidence) | |
| --- | --- | --- |
| % Mustard Husk | INTESTINAL | COLONIC |
| 0 | 11 ( 36%) | 18 ( 60%) |
| 2.5 | 10 ( 33%) | 8 ( 26%) |
| 5.0 | 9 ( 30%) | 6 ( 20%) |
| 7.5 | 3 ( 10%) | 1 ( 3%) |

\*    Accumulated data in 30 rats, 10 sacrificed at each of 26, 30 and 34 weeks following the first injection of the carcinogen DMH.

From Table 1 it can be seen that the number of rats with histologically (microscopic adenomas plus adenocarcinomas) confirmed gastrointestinal tract tumours fed a diet containing 20% lipids and 2.5, 5.0 or 7.5% untreated milled white mustard husk was lower than that of rats receiving a diet free of fibre.

Table 2 presents test data demonstrating the influence of untreated milled white mustard husk on the number of rats with metastasis following exposure to the carcinogen DMH.

## TABLE 2

| Experimental Groups<br>% Mustard Husk | No of rats* with metastasis (%) |
|:---:|:---:|
| 0 | 10 (100%) |
| 2.5 | 6 ( 60%) |
| 5.0 | 2 ( 20%) |
| 7.5 | 2 (20%) |

\* 10 rats per group sacrificed 26 weeks from the first injection of the carcinogen DMH.

From Table 2 it can be seen that the number of rats with metastasis fed a diet containing 20% lipids and 2.5, 5.0 or 7.5% untreated milled white mustard husk was greatly reduced compared to that of rats receiving a diet free of fibre.

The data clearly demonstrates the anticarcinogenic activity of untreated milled white mustard husk in rats fed a high lipid diet following exposure to the carcinogen DMH.

## Claims

1.  Use of untreated mustard husk for the preparation of a medicament for the prevention of colonic cancer.

2.  Use according to claim 1 characterised in that the untreated mustard husk is white or brown mustard husk.

3.  Use according to claim 1 characterised in that the untreated mustard husk is milled.

4.  Use according to claim 2 characterised in that the medicament is in unit dosage form, in the form of powders or granules packed into sachets, each unit containing from 0.5 to 5g preferably 1 to 3g of untreated mustard husk.

5.  Use according to claims 2 or claim 3 characterised in that the medicament is in unit dosage form in the form of tablets or capsules, each tablet or capsule containing 0.5 to 1.0g of untreated mustard husk.

## Patentansprüche

1.  Verwendung einer unbehandelten Senfschote zur Herstellung eines Medikaments zur Vermeidung von Kolonkrebs.

2.  Verwendung nach Anspruch 1,
    **dadurch gekennzeichnet,**
    daß die unbehandelte Senfschote eine weiße oder braune Senfschote ist.

3.  Verwendung nach Anspruch 1,
    **dadurch gekennzeichnet,**
    daß die unbehandelte Senfschote gemahlen ist.

**4.** Verwendung nach Anspruch 2,
**dadurch gekennzeichnet,**
daß das Medikament in Einheitsdosierungsform in Form von Pulvern oder Granalien ist, die in Packungen verpackt sind, wobei jede Einheit von 0,5 bis 5 g, vorzugsweise von 1 bis 3 g unbehandelte Senfschote enthält.

**5.** Verwendung nach Anspruch 2 oder 3,
**dadurch gekennzeichnet,**
daß das Medikament in Einheitsdosierungsform in Form von Tabletten oder Kapseln ist, wobei jede Tablette oder Kapsel 0,5 bis 1,0 g unbehandelte Senfschote enthält.

**Revendications**

1. Utilisation d'enveloppe ou gousse de moutarde non traitée pour la préparation d'un médicament destiné à la prévention d'un cancer du colon.

2. Utilisation selon la revendication 1, caractérisée en ce que la gousse de moutarde non traitée est de la gousse de moutarde blanche ou brune.

3. Utilisation selon la revendication 1, caractérisée en ce que la gousse de moutarde non traitée est moulue.

4. Utilisation selon la revendication 2, caractérisée en ce que le médicament est sous forme de doses unitaires, sous forme de poudres ou de granules emballées dans des sachets, chaque unité contenant de 0,5 à 5 g, de préférence 1 à 3 g de gousse de moutarde non traitée.

5. Utilisation selon la revendication 2 ou la revendication 3, caractérisée en ce que le médicament est sous forme de doses unitaires, sous forme de comprimés ou de capsules ou gélules, chaque comprimé ou chaque capsule ou gélule contenant 0,5 à 1,0 g d'enveloppe ou gousse de moutarde non traitée.